# EUROPEAN PATENT APPLICATION

(11) **EP 4 036 609 A1**
(43) Date of publication of application: **03.08.2022**
(21) Application number: 21154337.6
(22) Date of filing: 29.01.2021
(51) Int. Cl.: G01T 1/164, G01T 1/24

(54) **X-RAY SCATTER ESTIMATION**

(71) Applicant: Koninklijke Philips N.V., 5656 AG Eindhoven (NL)
(72) Inventor: LAHOUD, Elias, 5656 AE Eindhoven (NL)
(74) Representative: Philips Intellectual Property & Standards

(57) **Abstract**

An X-ray detector (100) includes a pixelated array (110) of X-ray detector elements (130_{i,j}) configured to determine detection times of received X-ray quanta from detection events representing the received X-ray quanta; and a processor (120) estimates an amount of scattered X-ray quanta received by the X-ray detector elements (130_{i,j}), based on a count of pairs of detection events detected within a predetermined time interval of each other by adjacent X-ray detector elements (130_{i,j}, 130ij(a..h)) in the pixelated array (110).

## Description

### TECHNICAL FIELD

The present disclosure relates to the estimation of scatter in an X-ray detector. An X-ray detector, a computed tomography imaging system that includes the X-ray detector, an X-ray imaging system that includes the X-ray detector, an X-ray scatter estimation method, an X-ray scatter correction method, a computer program product, and a computer-readable storage medium, are disclosed.

### BACKGROUND

X-ray imaging systems are employed in various medical imaging, baggage inspection, and materials analysis applications. Such imaging systems employ an X-ray detector that includes a pixelated array of X-ray detector elements. The detector elements detect X-ray quanta that are emitted by an X-ray source in order to generate X-ray images representing the attenuation of X-ray attenuating media disposed in an imaging region between the X-ray source and the X-ray detector. The spatial distribution of the detected X-ray quanta is used to reconstruct generate planar, or volumetric images. Various image reconstruction techniques are known for this purpose.

X-ray scatter is a process by which the directions and/or energies of X-rays emitted by the X-ray source are altered by the X-ray attenuating media in the imaging region. X-ray scatter results in degraded X-ray images. X-ray scatter often appears as a background haze in X-ray images and hampers image interpretation. X-ray scatter is explained in-part by the Compton effect, wherein elastic collisions between incident X-ray quanta and charged particles in the attenuating media result in a portion of the energy of incident X-ray quanta being transferred to the charged particles.

An X-ray anti-scatter grid is often used in X-ray imaging systems in order to reduce the effects of scatter. An anti-scatter grid may be provided by a one- or two-dimensional array of apertures defined in an X-ray absorbing material such as lead, tungsten and molybdenum. The X-ray anti-scatter grid is coupled to the X-ray radiation-receiving side of the X-ray detector, and its apertures limit the acceptance angle of the X-ray detector elements to X-ray quanta, thereby rejecting scattered X-ray quanta that have undergone significant path deviations.

X-ray scatter presents issues in both conventional X-ray detectors, as well as in spectral X-ray detectors. In contrast to conventional X-ray detectors which determine the amount of detected X-ray quanta within a single X-ray energy interval, spectral X-ray detectors determine the amount of detected X-ray quanta within multiple X-ray energy intervals. Spectral X-ray detectors have been employed in various spectral X-ray imaging systems and spectral computed tomography "CT" imaging systems, to generate planar X-ray images and volumetric X-ray images, respectively. The spectral images generated by such imaging systems can be used to discriminate between media that have similar X-ray attenuation values when measured within a single energy interval, and which would otherwise be indistinguishable in conventional X-ray images. Various dual- and multi-energy X-ray and CT imaging systems have been developed. For example, systems that employ temporally-sequential scanning with different energy X-rays, rapid kVp switching of the X-ray tube potential, multilayer detectors, dual X-ray sources, and photon counting detectors, have been developed for determining the amount of detected X-ray quanta within multiple X-ray energy intervals.

Estimates of the amount of scattered X-ray quanta have been used to compensate for X-ray scatter, and thereby improve the quality of images generated using X-ray detectors. For example, a document by Wiegert, J, et al., entitled "Impact of scattered radiation on spectral CT", Proc. SPIE 7258, Medical Imaging 2009: Physics of Medical Imaging, 72583X, 14 March 2009; https://doi.org/10.1117/12.813674, analyses the impact of scattered radiation in multi-energy CT quantitatively by means of Monte-Carlo simulation.

In spite of these developments, there remains room to improve the estimation of scatter in X-ray detectors.

### SUMMARY

According to one aspect of the present disclosure, an X-ray detector includes:
a pixelated array of X-ray detector elements configured to determine detection times of received X-ray quanta from detection events representing the received X-ray quanta; and
a processor configured to receive the detection times, and to estimate an amount of scattered X-ray quanta received by the X-ray detector elements, based on a count of pairs of detection events detected within a predetermined time interval of each other by adjacent X-ray detector elements in the pixelated array.

According to another aspect of the present disclosure, an X-ray scatter estimation method includes:
receiving detection times of detection events representing X-ray quanta received by a pixelated array of X-ray detector elements; and
estimating an amount of scattered X-ray quanta received by the X-ray detector elements, based on a count of pairs of detection events detected within a predetermined time interval of each other by adjacent X-ray detector elements in the pixelated array.

Further aspects, features and advantages of the present disclosure will become apparent from the following description of examples, which is made with reference to the accompanying drawings.

### BRIEF DESCRIPTION OF THE DRAWINGS

Fig. 1 is a schematic illustration of a pixelated array 110 of X-ray detector elements A1..A5 receiving a non-scattered X-ray quant P1, and a scattered X-ray quant S1.
Fig. 2 is a schematic illustration of an X-ray detector 100 including a pixelated array 110 of X-ray detector elements 130_{i,j}, and a processor 120, in accordance with some aspects of the present disclosure.
Fig. 3 is a graph illustrating normalized X-ray spectra generated by an X-ray detector in response to received X-ray quanta from a monochromatic 60 keV energy source, with different positions of an anti-scatter grid, "ASG".
Fig. 4 is a graph illustrating the ratio "P2T" between a count of the number of received X-ray quanta within the 60 keV photopeak (Energy ≥ 53 keV) of the spectra in Fig. 3, to a count of the number of received X-ray quanta within the tail of the spectra (20 keV ≤ Energy ≤ 53 keV) in Fig. 3 under different amounts of anti-scatter grid misalignment "ASG misalignment (mm)".
Fig. 5 is a schematic illustration of a computed tomography 200 imaging system, in accordance with some aspects of the present disclosure.
Fig. 6 is a flowchart illustrating an X-ray scatter estimation method, in accordance with some aspects of the present disclosure.

### DETAILED DESCRIPTION

Examples of the present disclosure are provided with reference to the following description and the figures. In this description, for the purposes of explanation, numerous specific details of certain examples are set forth. Reference in the specification to "an example", "an implementation" or similar language means that a feature, structure, or characteristic described in connection with the example is included in at least that one example. It is also to be appreciated that features described in relation to one example may also be used in another example, and that all features are not necessarily duplicated in each example for the sake of brevity. For instance, features described in relation to an X-ray detector may be implemented in an X-ray or CT imaging system, and in an X-ray scatter estimation method, and in an X-ray scatter correction method. Features disclosed in relation to these methods may be implemented in a computer program product, and in a processing arrangement, and in a system, in a corresponding manner.

In the following description, reference is made to an X-ray detector that is used to detect X-ray quanta. Reference is made to examples wherein the X-ray detector is a so-called direct detector wherein X-ray quanta are detected by collecting the electrical charges from charge clouds that are generated in a semiconductor material in response to the X-ray quanta being absorbed in the semiconductor material. Semiconductor materials such as cadmium telluride, CdTe, and cadmium zinc telluride "CZT", i.e. CdZnTe, are known for this purpose. However, it is also to be appreciated that examples in accordance with the present disclosure may also be used in so-called indirect detectors and wherein X-ray quanta are detected by using an optical detector to detect optical photons that are generated in a scintillator material in response to the X-ray quanta being absorbed in the scintillator material. Scintillator materials such as gadolinium oxysulfide "GOS", and caesium iodide, are known for this purpose.

Reference is made to examples wherein a planar X-ray detector is used in an X-ray imaging system to generate planar X-ray images. However, it is to be appreciated that the planar X-ray detector may also be used to detect X-rays in other types of imaging systems, such as for example a computed tomography "CT" imaging system, and from which volumetric or three-dimensional "3D" images, may be generated. It is also to be appreciated that the X-ray detector may be a linear X-ray detector, i.e. it may include a one-dimensional array of X-ray detector elements, rather than a planar, or two-dimensional array of X-ray detector elements. Moreover, whilst reference is made to the generation of planar images, it is to be appreciated that examples in accordance with the present disclosure may also be used with detectors that have various shapes, including curved detectors, and segmented detectors wherein multiple planar detectors are arranged around a curved surface.

Reference is also made to examples wherein the X-ray detector is used in a conventional X-ray imaging system, in other words, a system which determines the amount of detected X-ray quanta within a single X-ray energy interval. However, it is to be appreciated that X-ray detectors in accordance with the present disclosure may also be used in so-called spectral X-ray and spectral CT imaging systems which use detectors that determine the amount of detected X-ray quanta within multiple X-ray energy intervals.

Reference is also made to the use of an example X-ray detector in generating medical images. However, it is to be appreciated that detectors in accordance with the present disclosure may be used to generate other types of images, such as for example baggage scanning images and images for materials analysis.

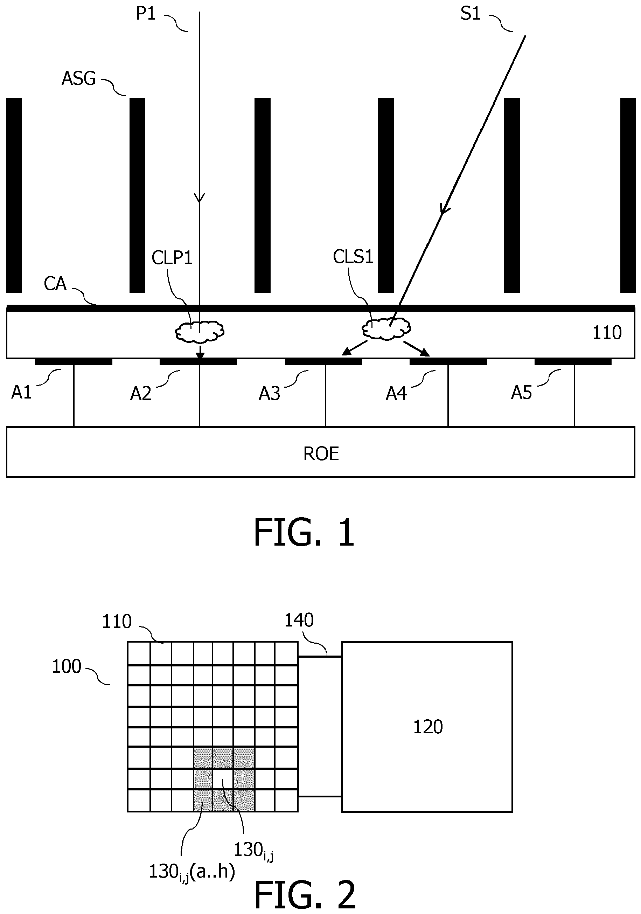

Fig. 1 is a schematic illustration of a pixelated array 110 of X-ray detector elements A1..A5 receiving a non-scattered X-ray quant P1, and a scattered X-ray quant S1. The X-ray detector illustrated in Fig. 1 represents a so-called direct detector, and wherein X-ray quanta are detected by collecting the electrical charges from charge clouds that are generated in a semiconductor material in response to the X-ray quanta being absorbed in the semiconductor material. The semiconductor material may for example be cadmium zinc telluride, or another material. In Fig. 1, a cathode CA is disposed on one face of the semiconductor material, and an array of anode contacts A1..A5 are disposed on an opposing face. The anode contacts A1..A5 define detector elements, or pixels, and thus define a pixelated array of X-ray detector elements. For ease of illustration, the illustrated array is one-dimensional, although a two-dimensional array may be provided in a similar manner. In-use, an electric field is applied between the anode contacts A1..A5 and the cathode CA, in order to separate the electrons and holes in the charge clouds that are generated in response to the X-ray quanta being absorbed in the semiconductor material. The electrons in the charge clouds are collected at the anodes contacts A1..A5. The electrons in each charge cloud are detected in the form of electrical current pulses at the anode contacts A1..A5, and may be measured by an electrical circuit. The electrical circuit may include an amplifier to amplify the current pulses and/or other electrical circuitry. With reference to Fig. 1, the electrical circuit may be included within read-out electronic circuit ROE.

The electrical pulses that are generated from the charge clouds in the semiconductor material in response to the received X-ray quanta, are examples of detection events that represent received X-ray quanta. These detection events are generated in semiconductor materials in so-called "direct detection" detectors. Optical pulses that are generated in a scintillator material in response to X-ray quanta that are received in a so-called indirect detection detector, are another example of detection events that represent received X-ray quanta. In an indirect detection detector, an optical detector is used to detect the pulse of optical photons that is generated each time an X-ray quant is absorbed in the scintillator material. The optical detector converts the optical pulse into an electrical pulse.

Various types of electrical circuit may be used to detect the electrical pulses that are generated by direct detection detectors, and by the optical detectors of indirect detection detectors. By way of some examples, a current amplifier, a charge amplifier, or an integrator circuit may be used. In one example, a so-called "photon counting" circuit is used, and wherein after an initial current threshold current is exceeded, each electrical pulse is counted in order to provide a total count value indicative of the total number of X-ray quanta that are received. The X-ray quanta may be counted within a predetermined period of time, such as an X-ray image frame period. In another type of photon-counting circuit, the amplitude of each electrical pulse, or the integrated value of each electrical pulse over time, is determined and/or compared with a one or more energy threshold values in order to further determine the energy of each received X-ray quant. The initial threshold current provides discrimination against noise and interference, and the energy threshold(s) are used to discriminate between multiple different X-ray energies. This latter type of photon counting detector is sometimes known as a spectral photon counting detector in view of its ability to determine the amount of detected X-ray quanta within multiple X-ray energy intervals.

Returning to Fig. 1, an X-ray anti-scatter grid ASG is coupled to the X-ray radiation-receiving side of the pixelated array. As mentioned above, an X-ray anti-scatter grid is used in X-ray imaging systems in order to reduce the effects of scatter by limiting the acceptance angle of the X-ray detector elements to X-ray quanta. The anti-scatter grid therefore allows the X-ray detector to reject X-ray quanta that are the result of scatter events wherein the X-ray quanta have undergone significant path deviations.

Improved rejection of scatter events may be achieved by increasing the length of the apertures of the anti-scatter grid ASG in Fig. 1. However, using longer apertures introduces other difficulties such as mechanical rigidity, alignment challenges, weight and cost. The ability of the X-ray anti-scatter grid to reject scatter events is also limited by materials constraints. The finite thickness of the apertures of the anti-scatter grid ASG in Fig. 1 that is necessary to stop off-axis X-ray quanta from travelling between neighboring apertures, also impacts the spacing of the anodes A1..A5, and thus the resolution of the pixelated array 110 that may be achieved. Consequently, a tradeoff between these constraints often results in an amount of scattered X-ray quanta being detected by the pixelated array 110.

The inventor has found that an estimate of the amount of scattered X-ray quanta received by a pixelated array of X-ray detector elements, such as the pixelated array 110 illustrated in Fig. 1, may be determined based on a count of pairs of detection events detected within a predetermined time interval of each other by adjacent X-ray detector elements in the pixelated array. With reference to Fig. 1, both the non-scattered X-ray quant P1, and the scattered X-ray quant S1, are within the acceptance angle of the anti-scatter grid ASG. The non-scattered X-ray quant P1, and the scattered X-ray quant S1, result in detection events in the form of charge clouds CLP1 and CLS1, respectively. The charge cloud CLP1 arising from the non-scattered X-ray quant P1 is generated at a position above anode A2, and the electrons therein are detected by anode A2 as a single electrical pulse. By contrast, the charge cloud CLS1 arising from scattered X-ray quant S1 is generated at a position between anode A3 and anode A4. In this situation it has been found that the charge cloud CLS1 splits, such that a first portion of the electrons in the charge cloud CLS1 are detected by anode A3, and a second portion of the electrons in the charge cloud CLS1 are detected by the adjacent anode A4. The first portion and the second portion result in the generation of near-simultaneous electrical pulses at adjacent anodes A3 and A4. These near-simultaneous electrical pulses are an example of a pair of detection events that are detected within a predetermined time interval of each other by adjacent X-ray detector elements, specifically anodes A3 and A4. Pairs of near-simultaneously detected events such as these, can each trigger a photon-counting detector to erroneously report that separate X-ray quants have been received, whereas in fact they are the result of the received X-ray quant having undergone a scattering event. This is because, as mentioned above, a photon counting detector is often triggered to record a count when the amplitude of the electrical pulse, or the integrated amplitude of the electrical pulse, exceeds an initial threshold.

In examples in accordance with the present disclosure, a count of the pairs of detection events detected within a predetermined time interval of each other by adjacent X-ray detector elements in the pixelated array, are used to estimate an amount of scattered X-ray quanta received by the X-ray detector elements. Thereto, Fig. 2 is a schematic illustration of an X-ray detector 100 including a pixelated array 110 of X-ray detector elements 130_{i,j}, and a processor 120, in accordance with some aspects of the present disclosure. With reference Fig. 1, the X-ray detector 100 includes:
a pixelated array 110 of X-ray detector elements 130_{i,j} configured to determine detection times of received X-ray quanta from detection events representing the received X-ray quanta; and
a processor 120 configured to receive the detection times, and to estimate an amount of scattered X-ray quanta received by the X-ray detector elements 130_{i,j}, based on a count of pairs of detection events detected within a predetermined time interval of each other by adjacent X-ray detector elements 130_{i,j}, 130ij(a..h) in the pixelated array 110.

The X-ray detector 100 illustrated in Fig. 2 may also include an anti-scatter grid (not illustrated) coupled to an X-ray radiation-receiving side of the pixelated array 110 of X-ray detector elements 130_{i,j}. In Fig. 2, the pixelated array 110 and the processor 120 in Fig. 2 are in communication with each other by means of a bus 140. It is noted that in general this communication may be any form of data communication, including wireless and wired data communication, and therefore the illustrated bus 140 is not essential.

In some examples, the use of direct detector materials in the X-ray detector 100 described above with reference to Fig. 2, is contemplated. In these examples, the X-ray detector elements 130_{i,j} include a semiconductor material, and the detection events comprise electrical pulses generated from charge clouds generated in the semiconductor material in response to the received X-ray quanta. However, the principles described above with reference to Fig.1, also apply to X-ray detector elements 130_{i,j} that include a scintillator material. When the pixelated array 110 in Fig. 1 includes a scintillator material, scintillation light pulses that are generated by scattered X-ray quanta at positions between pairs of adjacent X-ray detector elements, may likewise be detected in-part by each adjacent X-ray detector element in the pair. A first portion of a scintillation light pulse that is generated from a scattered X-ray quant may be detected by one optical detector pixel in the pair, and a second portion of the scintillation light pulse may be detected, near-simultaneously by the other, adjacent, optical detector pixel in the pair. Thus, in some examples, the use of direct detector materials in the X-ray detector 100 described above with reference to Fig. 2, is contemplated. In these examples, the X-ray detector elements 130_{i,j} includes a scintillator material, and the detection events comprise optical pulses generated in the scintillator material in response to the received X-ray quanta.

In the detector described with reference to Fig. 2, various timing circuits may be used to determine the detection times of received X-ray quanta from the detection events, i.e. the times of electrical pulses generated from charge clouds, or the times of optical pulses generated in the scintillator material, that represent the received X-ray quanta. In some examples, the detection times are determined by triggering a sampling of the value of a continuously-running timer when a characteristic of the detection event, such as the amplitude, or the time-integrated value, or the rate of change, of a detected electrical or optical pulse, meets a predetermined condition. For example, the value of the timer may be sampled if the amplitude of the detection event exceeds a predetermined threshold. The value of the timer may be sampled by, for example, storing the value of a continuously running timer into a register. In other examples, the detection times may be determined by triggering a counter to start counting clock pulses when a characteristic of the detection event, such as the amplitude, or the time-integrated value, or the rate of change, of a detected electrical or optical pulse, exceeds a predetermined threshold for a X-ray detector element, and storing an address defining the position of detection events from adjacent X-ray detector elements that occur within a predetermined number of clock pulses after the counter is triggered.

The processor 120 described with reference to Fig. 2 may for example be provided by an electronic circuit, such as for example an application specific integrated circuit, ASIC, and/or by a processor executing a set of instructions. The processor compares the detection times. The processor also determines a count of the number of pairs of detection events detected within a predetermined time interval of each other by adjacent X-ray detector elements in the array 110. In some examples, the predetermined time interval is less than or equal to 100 nanoseconds. In other examples, the predetermined time interval is less than or equal to 50 nanoseconds. In other examples, the predetermined time interval is less than or equal to 20 nanoseconds. In other examples, the predetermined time interval is less than or equal to 10 nanoseconds. In other examples, the predetermined time interval is less than or equal to 1 nanosecond. In other examples, the predetermined time interval is less than or equal to 100 picoseconds. In other examples, the predetermined time interval is less than or equal to 50 picoseconds. In so doing, a count of the pairs of near-simultaneous events which originate from the same detected X-ray quant, may be determined, whilst allowing for a margin of error to account for any differences in drift and diffusion times of the charge clouds in the semiconductor material between the adjacent detector elements, or to allow for any errors in determining the detection times of the received X-ray quanta for the adjacent detector elements, as may for example be caused by timing errors and differences in propagation delays of in the pixelated array, and so forth.

With reference to Fig. 2, an X-ray detector element 130_{i,j} has adjacent X-ray detector elements 130ij(a..h), which are illustrated in shaded form in Fig. 2. By way of an example, if, for example, the X-ray detector element 130_{i,j} generates an electrical pulse at a time T1 from a charge cloud generated in a semiconductor detector material in response to an X-ray quant being received, the processor then determines whether another detection event within any of its adjacent X-ray detector elements 130ij(a..h) has already occurred, or subsequently occurs, within the predetermined time interval of time T1. If so, the two events are deemed to be a pair of detection events, and the count of the pairs of detection events for the X-ray detector element 130_{i,j}, is incremented.

The same determination described in relation to the X-ray detector element 130_{i,j} is performed for multiple pixels in the pixelated array 110, in order to estimate a spatial distribution of an amount of scattered X-ray quanta received by the X-ray detector elements 130ij. It is noted that in general the amount may be estimated as a total count, or a rate, i.e. a flux, of scattered X-ray quanta, or a ratio of any of these quantities for the scattered X-ray quanta received by the X-ray detector elements 130_{i,j} to the X-ray quanta received by the X-ray detector elements 130_{i,j}. Moreover, the amount, being measured for X-ray detector elements 130_{i,j}, represents a spatial distribution. The amount may be determined within a predetermined time period, such as the time period of an X-ray image frame, in order to provide an amount for the X-ray image frame as a single image, or to provide an amount for an X-ray image frame within a temporal sequence of fluoroscopy images. The amount may alternatively be determined within another time period.

Having obtained an estimate of the amount of scattered X-ray quanta received by the X-ray detector elements 130_{i,j}, this estimate may be used to correct for scatter in the amount of X-ray quanta received by the X-ray detector elements 130_{i,j}. In some examples, the processor 120 may further compute an amount of X-ray quanta received by the X-ray detector elements 130_{i,j}, and correct for scatter in the amount of X-ray quanta received by the X-ray detector elements 130_{i,j} based on the estimated amount of scattered X-ray quanta received by the X-ray detector elements 130_{i,j}. The amount of X-ray quanta received by the X-ray detector elements 130_{i,j} includes both scattered and non-scattered X-ray quanta. As with the aforementioned estimate, the amount of X-ray quanta received by the X-ray detector elements 130_{i,j} may be a total count, or a rate, i.e. a flux, of received X-ray quanta. Likewise, since the amount is measured for X-ray detector elements 130_{i,j}, it also represents a spatial distribution. The amount of X-ray quanta received by the X-ray detector elements 130_{i,j}, and the estimate of the amount of scattered X-ray quanta received by the X-ray detector elements 130_{i,j}, may advantageously be measured within the same predetermined time period. Consequently, the estimate provides an accurate estimate of the scatter, because the X-ray attenuating media in the imaging region that give rise to the scatter, is present when both spatial distributions are determined.

In one example, the processor may correct for scatter in the amount of X-ray quanta received by the X-ray detector elements 130_{i,j}, by subtracting the spatial distribution of the estimate of the amount of scattered X-ray quanta received by the X-ray detector elements 130_{i,j}, from the spatial distribution of the amount of X-ray quanta received by the X-ray detector elements 130_{i,j}. The subtraction may be performed for corresponding X-ray detector elements 130_{i,j} in the pixelated array 110. In this example the processor corrects for scatter in the amount of X-ray quanta received by the X-ray detector elements 130_{i,j}, by, for at least some of the X-ray detector elements 130_{i,j}: subtracting the estimated amount of scattered X-ray quanta received by the X-ray detector element, from the computed amount of X-ray quanta received by the X-ray detector element.

In another example, the processor may correct for scatter by subtracting an average estimated amount of scattered X-ray quanta received by the X-ray detector elements from the computed amount of X-ray quanta received by the X-ray detector elements. In this example the processor corrects for scatter in the amount of X-ray quanta received by the X-ray detector elements 130_{i,j}, by, for at least some of the X-ray detector elements 130_{i,j}: computing an average estimated amount of scattered X-ray quanta received by the X-ray detector element based on the estimated amount of scattered X-ray quanta received by the X-ray detector element and one or more neighboring X-ray detector elements, and subtracting the average estimated amount of scattered X-ray quanta received by the X-ray detector element from the computed amount of X-ray quanta received by the X-ray detector element. In this example, the average estimated amount of scattered X-ray quanta received by the X-ray detector element, such as X-ray detector element 130_{i,j} in in Fig. 2, may be computed as an average of the estimated amount of scattered X-ray quanta received by the X-ray detector element 130_{i,j} and the eight neighboring X-ray detector elements 130ij(a..h).

As mentioned above, the estimate of the amount of scattered X-ray quanta received by the X-ray detector elements 130_{i,j}, and the amount of X-ray quanta received by the X-ray detector elements 130_{i,j}, may be detected in a predetermined time period, such as an image frame. The image frame may form part of a temporal sequence of image frames, such as the image frames generated during fluoroscopy imaging. The detection times of received X-ray quanta may thus also be determined within a predetermined time period. In some examples, the array of X-ray detector elements 130_{i,j} determines the detection times of received X-ray quanta within each of a plurality of time periods, each time period defining the acquisition of an X-ray image frame in a temporal sequence of X-ray image frames; and the processor corrects for scatter in each image frame by adjusting the amount of X-ray quanta received by the X-ray detector elements 130_{i,j} in the image frame based on the estimated amount of scattered X-ray quanta received by the X-ray detector elements 130_{i,j} in the image frame and/or in one or more previous image frames.

Thus, in one example, the adjusting may include subtracting the estimated amount of scattered X-ray quanta received by the X-ray detector elements 130_{i,j} in a current image frame from the amount of X-ray quanta received by the X-ray detector elements 130_{i,j} in the current image frame.

In another example, the estimated amount of scattered X-ray quanta received by the X-ray detector elements 130_{i,j} may be determined every N image frames, and the estimate used to correct for scatter in the image frame for which the scatter was estimated, as well as for the following N-1 image frames. In so doing, the complexity of the pixelated array 110 may be reduced due the need to determine detection times less frequently. The amount of processing performed by the processor 120 to estimate and correct for scatter, may also be reduced.

In yet another example, a rolling average of the estimated amount of scattered X-ray quanta received by the X-ray detector elements 130_{i,j} may be determined for the current image frame and the previous N-1 image frames, and the rolling average for these N image frames used to correct for scatter in the current image frame. Combinations of these approaches may also be used to advantageous effect.

Having performed the above correction for scatter, the processor 120 may reconstruct a volumetric or planar image based on the adjusted amount of X-ray quanta received by the X-ray detector elements 130_{i,j} in each image frame. In so doing, the scatter correction may improve the image quality in the reconstructed images. Various planar and volumetric reconstruction techniques are known for this purpose.

In some examples, the pixelated array 110 of X-ray detector elements 130_{i,j} also determines the energy values of the received X-ray quanta. In accordance with these examples, the pixelated array 110 of X-ray detector elements 130_{i,j} is further configured to determine corresponding energy values of the received X-ray quanta; and the processor 120 is further configured to receive the energy values, and to estimate the amount of scattered X-ray quanta received by the X-ray detector elements 130_{i,j} based further on the energy values.

The energy values of the received X-ray quanta may be determined using a spectral X-ray detector as the X-ray detector 100 in Fig. 2. As mentioned above, in contrast to conventional X-ray detectors which determine the amount of detected X-ray quanta within a single X-ray energy interval, spectral X-ray detectors determine the amount of detected X-ray quanta within multiple X-ray energy intervals. Various dual- and multi-energy X-ray detectors have been developed. For example, systems that employ temporally-sequential scanning with different energy X-rays, rapid kVp switching of the x-ray tube potential, multilayer detectors, dual X-ray sources, and photon counting detectors, have been developed for determining the amount of detected X-ray quanta within multiple X-ray energy intervals.

In one example, a photon counting spectral X-ray detector may be used to determine the energy values of the received X-ray quanta. Continuing from the above example wherein the detection times of received X-ray quanta are determined by triggering a sampling of the value of a continuously-running timer, or by triggering a counter, when a characteristic of the detection event, such as the amplitude, or the time-integrated value, or the rate of change of a detected electrical or optical pulse, meets a predetermined condition, in this example the same trigger may also be used to trigger a determination of the energy values of the received X-ray quanta. The same trigger may for example be used to trigger an integrator to integrate over time the electrical pulse that is generated from each charge cloud in the semiconductor material in response to the received X-ray quant. The result of this integration represents the energy of the X-ray quant. The energy of the received X-ray quant within one of multiple energy intervals may be determined by comparing the result of the integration with one or more energy threshold values. A single energy threshold value may for example be used to distinguish between two energy intervals. The threshold to the triggering provides discrimination against false triggers from sources such as noise and interference, and the single energy threshold provides a reliable distinction between two energy intervals. Additional energy thresholds may also be provided in a similar manner in order to distinguish between further energy intervals.

In a similar manner, rather than triggering an integrator to integrate over time the electrical pulse that is generated from each charge cloud in the semiconductor material in response to the received X-ray quant, instead, the amplitude of the electrical pulse may be compared with one or more energy threshold values. The peak amplitude of the electrical pulse is indicative of the energy of the received X-ray quant. The energy of the received X-ray quant within one of multiple energy intervals may therefore be determined by comparing amplitude of the electrical pulse with one or more energy threshold values.

Thus, in some examples the X-ray detector elements 130_{i,j} include a semiconductor material, and the detection events comprise electrical pulses generated from charge clouds generated in the semiconductor material in response to the received X-ray quanta, and the charge clouds are detected as electrical pulses; and the energy value of each received X-ray quant is determined based on an amplitude of each detected electrical pulse, or based on an integral of each detected electrical pulse over time.

Likewise, if the photon counting spectral X-ray detector employs a scintillator material, i.e. an indirect detection detector, the electrical pulses that are generated from the optical pulses in the scintillator material by the optical detectors, may processed in a similar manner in order to determine the energy values of the received X-ray quanta.

Thus, in some examples, the X-ray detector elements 130_{i,j} include a scintillator material, and the detection events comprise optical pulses generated in the scintillator material in response to the received X-ray quanta, and the optical pulses are detected by a photodetector as electrical pulses; and the energy value of each received X-ray quant is determined based on an amplitude of each detected electrical pulse, or based on an integral of each detected electrical pulse over time.

In some examples, processor 120 uses the energy values of the received X-ray quanta to improve the estimate of the amount of scattered X-ray quanta received by the X-ray detector elements 130_{i,j}.

For example, the count of pairs of detection events detected within a predetermined time interval of each other by adjacent X-ray detector elements 130_{i,j}, 130ij(a..h) described above may include some false-positive pairs which are result of near-simultaneously detected events from separate X-ray quanta, rather than the result of a scattering event. These false-positive pairs may be distinguished based on the energy values that are determined for the two quanta in the pair. For example, if, based on the X-ray source energy, the maximum expected energy from any quant is 60 keV, false positive pairs may be defined as pairs wherein the quanta in the pair both have an energy that exceeds a predetermined value, such as 55 keV, or as pairs wherein the quanta in the pair both have an energy that is within a predetermined range, such as 55 - 60 keV. False positive pairs that are identified in this manner may be re-assigned as actual X-ray quanta received by the X-ray detector elements 130_{i,j}, rather than scattered X-ray quanta. This improves the estimate of the amount of scattered X-ray quanta received by the X-ray detector elements 130_{i,j}.

By way of another example, and with reference to Fig. 1, some scattering events that result in the charge cloud, or likewise the scintillation light pulse, splitting into a first portion and a second portion that are detected by adjacent anodes A3, A4, or adjacent optical detector pixels, such that their total energy equates to an expected value. For example, if the X-ray source is a monochromatic 60 keV source, the total energy detected by adjacent anodes or pixels may be expected to equate to 60 keV. Therefore, by summing the energy values of each pair of detection events that are detected within a predetermined time interval of each other by adjacent X-ray detector elements 130_{i,j}, 130_{i,j}(a..h), and comparing this summed energy with an energy value, such as the expected 60 keV, it may be determined whether the detection events originated from a scatter event, such as scattered X-ray quant S1 in Fig. 1.

Thus, in some examples, the count of pairs of detection events comprises a count of pairs of detection events having energy values that exceed a predetermined threshold energy value, or a count of pairs of detection events having energy values that are within a predetermined range.

In order to illustrate the above principles wherein the electrons from charge clouds that are generated at positions between pairs of adjacent X-ray detector elements, are detected in-part by each of the adjacent X-ray detector elements in the pair, an experiment was performed to determine the sensitivity of a photon counting X-ray detector to anti-scatter grid misalignment. In the experiment, a "direct" photon counting X-ray detector was irradiated with X-ray quanta from a 60 keV monochromatic X-ray source with the anti-scatter grid in three positions: i) no anti-scatter grid, ii) anti-scatter grid correctly aligned with the pixels in the pixelated array of X-ray detector elements, and iii) anti-scatter grid completely misaligned. The energy spectrum of the photon counting detector illustrates that the electrons from charge clouds that are generated by X-ray quanta from the monochromatic X-ray source at positions between pairs of adjacent X-ray detector elements, are split, such that their electrons are detected by both of the adjacent X-ray detector pixels, particularly when the anti-scatter grid is misaligned. It is noted that this experiment was performed purely to illustrate the effect of charge cloud splitting, and that the monochromatic X-ray source and energy discriminating X-ray detector are not essential to the embodiments disclosed herein. Nor are the principles demonstrated by the experiment limited to the example 60 keV X-ray energy used in the experiment. Rather, embodiments in accordance with the present disclosure may be used with monochromatic, or polychromatic, i.e. "broadband" X-ray sources, and with conventional, i.e. non energy-discriminating X-ray detectors, or with energy-discriminating detectors, as well as with indirect detection X-ray detectors rather than the example direct detection detector, and with different X-ray energy values to the example 60 keV.

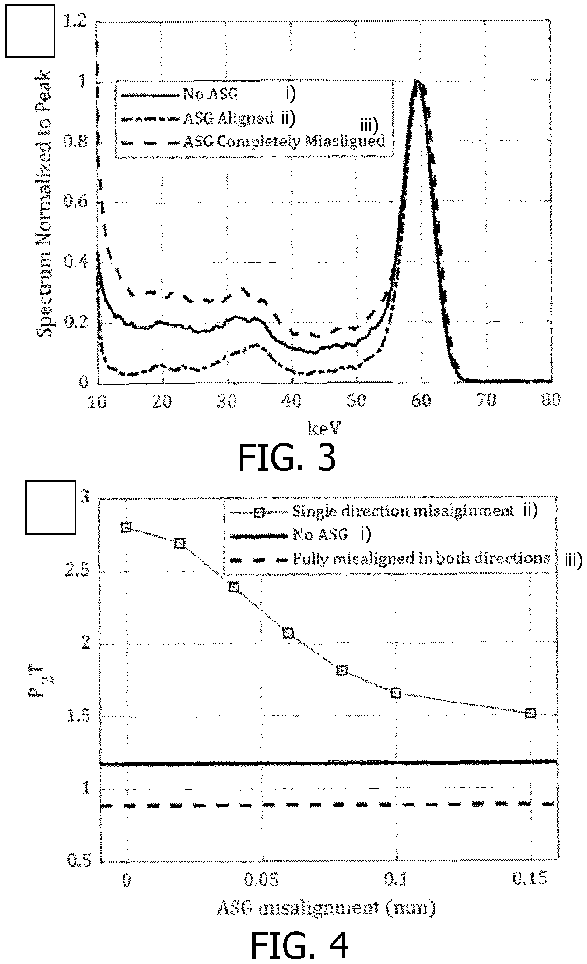

Thereto, Fig. 3 is a graph illustrating normalized X-ray spectra generated by an X-ray detector in response to X-ray quanta received from a monochromatic 60 keV energy source, with different positions of an anti-scatter grid, "ASG". Fig. 4 is a graph illustrating the ratio "P2T" between a count of the number of received X-ray quanta within the 60 keV photopeak (Energy ≥ 53 keV) of the spectra in Fig. 3, to a count of the number of received X-ray quanta within the tail of the spectra (20 keV ≤ Energy ≤ 53 keV) in Fig. 3 under different amounts of anti-scatter grid misalignment "ASG misalignment (mm)".

The X-ray detector used to obtain the graphs in Fig. 3 and Fig. 4 was a Philips Spectral Photon Counting CT prototype detector. Each detector tile in the detector is formed from a single crystal of CdZnTe with dimensions of 16 mm × 11 mm × 2 mm mounted on a Philips ChromAIX2 chip with five paralyzable counters. The anode surface of the CZT is pixelated with pixel pad size of 0.4 mm and pixel pitch of 0.5 mm for a total of 32 × 22 pixels. The X-ray detector's energy thresholds were energy calibrated, and can be set to count photons above any threshold energy in the range of 0 to 160 keV with an energy resolution of 0.5 keV/LSB. The energy resolution of the detector as measured by monochromatic sources was 8 keV FWHM. The anti-scatter grid was made of Tungsten with 0.5 mm pitch and 10 mm wall height, and wall thickness of approximately 90 µm. The X-ray source used to obtain the graphs was a monochromatic 59.5 keV Americium-241 source with 50 mCi activity, and was placed 900 mm away from the detector.

Turning to Fig. 3, the solid curve represents the X-ray spectrum of the received X-ray quanta in the situation when there is i) no anti-scatter grid. The peak of the spectrum is at approximately 60 keV, i.e. the expected energy of non-scattered monochromatic 60 keV X-ray quanta. The finite bandwidth of the monochromatic X-ray source illustrates that the X-ray quanta have energy values with a full width half maximum of approximately 6 keV. However, due to the absence of an anti-scatter grid in situation i), a significant proportion of the received X-ray quanta are received by the X-ray detector in the channels that separate the individual detector elements, or pixels. With reference to Fig. 1, the channels are the spaces between the anodes A1..A5. Similar spaces exist in an orthogonal direction, and in diagonal directions, in a 2D array of detector elements. Consequently, as explained above with reference to Fig. 1, the resulting charge clouds that are generated between a pair of adjacent detector elements, may split such that portions of the electrons in each charge cloud are detected by both of the adjacent detector elements in the pair. Returning to Fig. 3, the effect of the charge cloud splitting is seen in the significant "tail" of the spectrum in Fig. 3, specifically at energy values in the range from 20 keV to 53 keV. Similar results may also be expected from other X-ray source and X-ray detector arrangements to the one described above that was used to obtain the results in Fig. 3. In Fig. 4, the corresponding graph, with a solid line, of the ratio "P2T" with i) no anti-scatter grid, is clearly invariant under small anti-scatter grid misalignment values "ASG misalignment (mm)" since there is no anti-scatter grid. It is noted that the absolute value of the "P2T" ratio with no scatter grid is in-part dependent on the ratio of the total area of the channels to the total area of the detector elements.

In Fig. 3, under situation iii), i.e. the anti-scatter grid is completely misaligned, an even greater proportion of the total X-ray quanta are identifiable in the "tail" of the spectrum than in the absence of an anti-scatter grid. Consequently, the corresponding graph in Fig. 4 of the ratio "P2T" with the anti-scatter grid completely misaligned, is lower than in the absence of an anti-scatter grid. The ratio "P2T" with iii) the anti-scatter grid completely misaligned, is again invariant under small anti-scatter grid misalignment values - in this situation due to its gross misalignment.

In Fig. 3, under situation ii), i.e. the anti-scatter grid is correctly aligned with the pixelated array of X-ray detector elements. The peak in the spectrum is at the expected 60 keV energy of the monochromatic X-ray quanta, but a significant proportion of the total X-ray quanta are still identifiable in the "tail" of the spectrum. This tail is relatively lower than in situation i) and situation iii), because in situation ii) the received X-ray quanta are largely blocked by the anti-scatter grid from being received in the channels. However, the graph in situation ii) indicates that even in the presence of an anti-scatter grid, some of the received X-ray quanta are indeed received in the channels, and that portions of their charge clouds, or detection events, are subject to being detected by adjacent detector elements. The corresponding graph in Fig. 4 of the ratio "P2T" under situation ii), i.e. with the anti-scatter grid correctly aligned with the pixelated array of X-ray detector elements and with an "ASG misalignment (mm)" of zero, indicates the highest ratio "P2T" of situations i) - iii). This is expected since the purpose of the anti-scatter grid is in-part to block X-ray quanta from being received in the channels. Moreover, the graph in Fig. 4 illustrates a significant variation in the ratio "P2T" with a single-direction misalignment of the anti-scatter grid. As the anti-scatter grid misalignment is increased, i.e. "ASG misalignment (mm)" increases, an increased proportion of the received X-ray quanta are received in the increasingly-exposed channels, which increases the proportion of charge clouds that are split, and whose electrons are thus detected in-part by adjacent X-ray detector elements. This reduces the ratio "P2T".

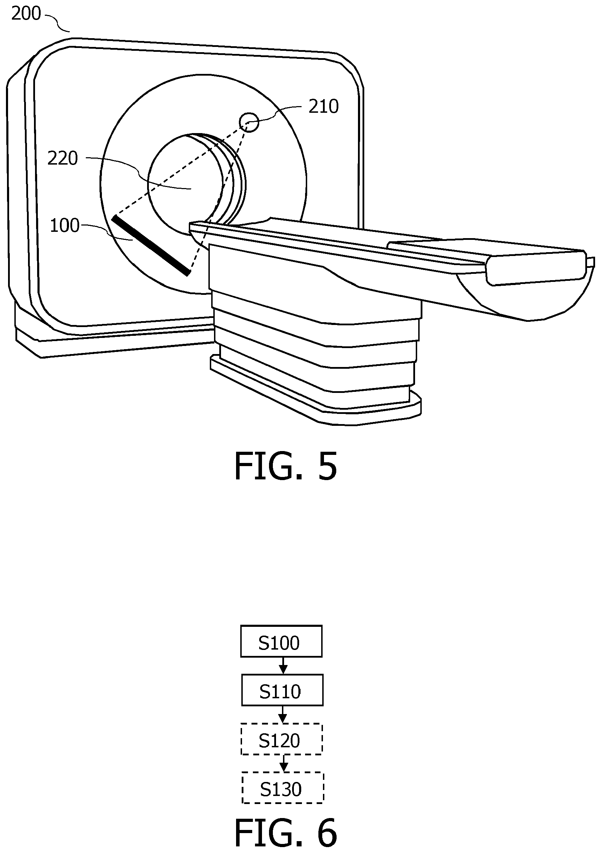

In some examples, the X-ray detector 100 described above with reference to Fig. 2 is included in an imaging system. The imaging system may for example be a computed tomography imaging system, or an X-ray imaging system. Thereto, Fig. 5 is a schematic illustration of a computed tomography 200 imaging system, in accordance with some aspects of the present disclosure. The computed tomography 200 imaging system includes the X-ray detector 100 described above, and an X-ray source 210. The X-ray source 210 and the X-ray detector are separated by an imaging region 220 in order to generate X-ray images representing attenuation of X-rays passing through the imaging region 220 between the X-ray source 210 and the X-ray detector 100. During imaging, the X-ray source 210 and the X-ray detector 100 illustrated in Fig. 5 are rotated around the imaging region 220 whilst generating projection image data. The image data is then reconstructed using known image reconstruction techniques to provide a 3D image representing X-ray attenuation in the imaging region 220. In some examples, the X-ray detector 100 includes a one-dimensional pixelated array of X-ray detector elements, and in other examples, the X-ray detector 100 includes a two-dimensional pixelated array of X-ray detector elements. The imaging system may be provided with a patient bed, as also illustrated in Fig. 5.

An X-ray scatter estimation method, and a related X-ray scatter correction method, are also provided in accordance with further examples of the present disclosure. Thereto, Fig. 6 is a flowchart illustrating an X-ray scatter estimation method, in accordance with some aspects of the present disclosure.

With reference to Fig. 6, the X-ray scatter estimation method, includes:
receiving S100 detection times of detection events representing X-ray quanta received by a pixelated array of X-ray detector elements 130_{i,j}; and
estimating S110 an amount of scattered X-ray quanta received by the X-ray detector elements 130_{i,j}, based on a count of pairs of detection events detected within a predetermined time interval of each other by adjacent X-ray detector elements 130_{i,j}, 130_{i,j}(a..h) in the pixelated array 110.

With further reference to Fig. 6, the X-ray scatter correction method includes:
receiving S100 detection times of detection events representing X-ray quanta received by a pixelated array of X-ray detector elements 130_{i,j};
computing S120 an amount of detected X-ray quanta detected by the pixelated array 110;
estimating S110 an amount of scattered X-ray quanta received by the X-ray detector elements 130_{i,j}, based on a count of pairs of detection events detected within a predetermined time interval of each other by adjacent X-ray detector elements 130_{i,j}, 130ij(a..h) in the pixelated array 110; and
correcting S130 the amount of detected X-ray quanta, with the estimated amount of scattered X-ray quanta received by the X-ray detector elements 130_{i,j}.

Aspects of the X-ray scatter estimation method and the X-ray scatter correction method may be implemented by an ASIC. However, it is also noted that aspects of the methods disclosed herein may also be implemented by a computer. The methods may be provided as a non-transitory computer-readable storage medium including computer-readable instructions stored thereon which, when executed by at least one processor, cause the at least one processor to perform the method. In other words, the methods may be implemented in a computer program product. The computer program product can be provided by dedicated hardware or hardware capable of running the software in association with appropriate software. When provided by a processor or "processing arrangement", the functions of the method features can be provided by a single dedicated processor, by a single shared processor, or by a plurality of individual processors, some of which can be shared. The explicit use of the terms "processor" or "controller" should not be interpreted as exclusively referring to hardware capable of running software, and can implicitly include, but is not limited to, digital signal processor "DSP" hardware, read only memory "ROM" for storing software, random access memory "RAM", a non-volatile storage device, and the like. Furthermore, examples of the present disclosure can take the form of a computer program product accessible from a computer usable storage medium or a computer-readable storage medium, the computer program product providing program code for use by or in connection with a computer or any instruction execution system. For the purposes of this description, a computer-usable storage medium or computer-readable storage medium can be any apparatus that can comprise, store, communicate, propagate, or transport a program for use by or in connection with an instruction execution system, apparatus, or device. The medium can be an electronic, magnetic, optical, electromagnetic, infrared, or semiconductor system or device or propagation medium. Examples of computer-readable media include semiconductor or solid-state memories, magnetic tape, removable computer disks, random access memory "RAM", read only memory "ROM", rigid magnetic disks, and optical disks. Current examples of optical disks include compact disk-read only memory "CD-ROM", optical disk-read/write "CD-R/W", Blu-Ray^{™}, and DVD

The above examples are to be understood as illustrative of the present disclosure and not restrictive. Further examples are also contemplated. For instance, the examples described in relation to the method, may also be provided by a computer program product, or by a computer-readable storage medium, or by a processing arrangement, or by a system, in a corresponding manner. It is to be understood that a feature described in relation to any one example may be used alone, or in combination with other described features, and may also be used in combination with one or more features of another of the examples, or a combination of other examples. Furthermore, equivalents and modifications not described above may also be employed without departing from the scope of the invention, which is defined in the accompanying claims. In the claims, the word "comprising" does not exclude other elements or operations, and the indefinite article "a" or "an" does not exclude a plurality. The mere fact that certain features are recited in mutually different dependent claims does not indicate that a combination of these features cannot be used to advantage. Any reference signs in the claims should not be construed as limiting their scope.

## Claims

1. An X-ray detector (100) comprising:
a pixelated array (110) of X-ray detector elements (130_{i,j}) configured to determine detection times of received X-ray quanta from detection events representing the received X-ray quanta; and
a processor (120) configured to receive the detection times, and to estimate an amount of scattered X-ray quanta received by the X-ray detector elements (130_{i,j}), based on a count of pairs of detection events detected within a predetermined time interval of each other by adjacent X-ray detector elements (130_{i,j}, 130_{i,j}(a..h)) in the pixelated array (110).

2. The X-ray detector according to claim 1, wherein the predetermined time interval is less than or equal to 100 nanoseconds.

3. The X-ray detector according to claim 1, wherein the X-ray detector elements (130_{i,j}) comprise a semiconductor material, and wherein the detection events comprise electrical pulses generated from charge clouds generated in the semiconductor material in response to the received X-ray quanta; or
wherein the X-ray detector elements (130_{i,j}) comprise a scintillator material, and wherein the detection events comprise optical pulses generated in the scintillator material in response to the received X-ray quanta.

4. The X-ray detector according to any one of claims 1 - 3, wherein the pixelated array (110) of X-ray detector elements (130_{i,j}) is further configured to determine corresponding energy values of the received X-ray quanta; and wherein the processor (120) is further configured to receive the energy values, and to estimate the amount of scattered X-ray quanta received by the X-ray detector elements (130_{i,j}) based further on the energy values.

5. The X-ray detector according to claim 4, wherein i) the X-ray detector elements (130_{i,j}) comprise a semiconductor material, and wherein the detection events comprise electrical pulses generated from charge clouds generated in the semiconductor material in response to the received X-ray quanta, and wherein the charge clouds are detected as electrical pulses, or ii) wherein the X-ray detector elements (130_{i,j}) comprise a scintillator material, and wherein the detection events comprise optical pulses generated in the scintillator material in response to the received X-ray quanta, and wherein the optical pulses are detected by a photodetector as electrical pulses, respectively; and
wherein the energy value of each received X-ray quant is determined based on an amplitude of each detected electrical pulse, or based on an integral of each detected electrical pulse over time.

6. The X-ray detector according to claim 4, wherein the count of pairs of detection events comprises a count of pairs of detection events having energy values that exceed a predetermined threshold energy value, or a count of pairs of detection events having energy values that are within a predetermined range.

7. The X-ray detector according to claim 1, wherein the processor is further configured to compute an amount of X-ray quanta received by the X-ray detector elements (130_{i,j}), and to correct for scatter in the amount of X-ray quanta received by the X-ray detector elements (130_{i,j}) based on the estimated amount of scattered X-ray quanta received by the X-ray detector elements (130_{i,j}).

8. The X-ray detector according to claim 7, wherein the processor is configured to correct for scatter in the amount of X-ray quanta received by the X-ray detector elements (130_{i,j}), by, for at least some of the X-ray detector elements (130_{i,j}):
subtracting the estimated amount of scattered X-ray quanta received by the X-ray detector element, from the computed amount of X-ray quanta received by the X-ray detector element; or
computing an average estimated amount of scattered X-ray quanta received by the X-ray detector element based on the estimated amount of scattered X-ray quanta received by the X-ray detector element and one or more neighboring X-ray detector elements, and subtracting the average estimated amount of scattered X-ray quanta received by the X-ray detector element from the computed amount of X-ray quanta received by the X-ray detector element.

9. The X-ray detector according to claim 7 or claim 8, wherein the array of X-ray detector elements (130_{i,j}) is configured to determine the detection times of received X-ray quanta within each of a plurality of time periods, each time period defining the acquisition of an X-ray image frame in a temporal sequence of X-ray image frames;
wherein the processor is configured to correct for scatter in each image frame by adjusting the amount of X-ray quanta received by the X-ray detector elements (130_{i,j}) in the image frame based on the estimated amount of scattered X-ray quanta received by the X-ray detector elements (130_{i,j}) in the image frame and/or in one or more previous image frames.

10. The X-ray detector according to claim 9, wherein the processor is further configured to reconstruct a volumetric or planar image based on the adjusted amount of X-ray quanta received by the X-ray detector elements (130_{i,j}) in each image frame.

11. The X-ray detector according to claim 1, wherein the processor comprises an electronic circuit, or an application specific integrated circuit, ASIC.

12. A computed tomography (200) or X-ray imaging system comprising the X-ray detector (100) according to any one of claims 1 - 11; and an X-ray source (210); and
wherein the X-ray source (210) and the X-ray detector are separated by an imaging region (220) for generating X-ray images representing attenuation of X-rays passing through the imaging region (220) between the X-ray source (210) and the X-ray detector (100).

13. An X-ray scatter estimation method, comprising:
receiving (S100) detection times of detection events representing X-ray quanta received by a pixelated array (110) of X-ray detector elements (130_{i,j}); and
estimating (S110) an amount of scattered X-ray quanta received by the X-ray detector elements (130_{i,j}), based on a count of pairs of detection events detected within a predetermined time interval of each other by adjacent X-ray detector elements (130_{i,j}, 130_{i,j}(a..h)) in the pixelated array (110).

14. An X-ray scatter correction method, comprising:
receiving (S100) detection times of detection events representing X-ray quanta received by a pixelated array (110) of X-ray detector elements (130_{i,j});
computing (S120) an amount of detected X-ray quanta detected by the pixelated array (110);
estimating (S110) an amount of scattered X-ray quanta received by the X-ray detector elements (130_{i,j}), based on a count of pairs of detection events detected within a predetermined time interval of each other by adjacent X-ray detector elements (130_{i,j}, 130_{i,j}(a..h)) in the pixelated array (110); and
correcting (S130) the amount of detected X-ray quanta, with the estimated amount of scattered X-ray quanta received by the X-ray detector elements (130_{i,j}).

15. A computer program product comprising instructions which when executed by one or more processors, cause the one or more processors to carry the method according to any one of claims 12 - 14.
